# EUROPEAN PATENT SPECIFICATION

(11) **EP 0 598 029 B1**
(45) Date of publication and mention of the grant of the patent: **27.02.2002**
(21) Application number: 92917724.4
(22) Date of filing: 06.08.1992
(51) Int. Cl.: C12N 15/86, C12N 5/10

(54) **RETROVIRAL VECTORS CONTAINING INTERNAL RIBOSOME ENTRY SITES**
INTERNE RIBOSOM EINTRITTSSTELLEN ENTHALTENE RETROVIRALE VEKTOREN
VECTEURS RETROVIRAUX CONTENANT DES SITES INTERNES D'ENTREE DE RIBOSOME

(30) Priority: 07.08.1991 US 741740
(43) Date of publication of application: 25.05.1994
(73) Proprietor: ANDERSON, W., French, Bethesda, MD 20817 (US); MORGAN, Richard, A., Elkridge, MD 21227 (US); COUTURE, Larry, Silver Springs, MD 20902 (US)
(72) Inventor: ANDERSON, W., French, Bethesda, MD 20817 (US); MORGAN, Richard, A., Elkridge, MD 21227 (US); COUTURE, Larry, Silver Springs, MD 20902 (US)
(74) Representative: Becker, Konrad
(86) International application number: US9206572
(87) International publication number: WO9303143

(56) References cited:
- WO-A-90/01550
- MOL. CELL. BIOL., vol. 8, no. 4, April 1988 ASM WASHINGTON, DC,US, pages 1803-1808, R.W. OVERELL ET AL. 'Stably transmitted triple-promoter retroviral vectors and their use in transformation of primary mammalian cells'
- NATURE, vol. 353, 5 September 1991 MACMILLAN JOURNALS LTD., LONDON,UK, pages 90-94, D.G. MACEJAK AND P. SARNOW 'Internal initiation of translation mediated by the 5' leader of a cellular mRNA'
- J. VIROLOGY, vol. 65, no. 9, September 1991 AM.SOC.MICROBIOL.,WASHINGTON,US, pages 4985-4990, M.A. ADAM ET AL. 'Internal initiation of translation in retroviral vectors carrying Picornavirus 5'nontranlated regions'
- MOL. CELL. BIOL., vol. 11, no. 12, December 1991 ASM WASHINGTON, DC,US, pages 5848-5859, I.R. GHATTAS ET AL. 'The Encephalomyocarditis virus internal ribosome entry site allows efficient coexpression of two genes from a recombinant provirus in cultured cells and in embryos'
- VIROLOGY, vol. 186, no. 2, February 1992 ACADEMIC PRESS, INC.,NEW YORK, US, pages 669-675, H.-M. KOO ET AL. 'A spleen necrosis virus-based retroviral vector which expresses two genes from a dicistroninc mRNA '
- NUCLEIC ACIDS RESEARCH, vol. 20, no. 6, 25 March 1992 IRL PRESS LIMITED,OXFORD,ENGLAND, pages 1293-1299, R.A. MORGEN ET AL. 'Retroviral vectors containing putative internal ribosome entry sites: development of a polycistroninc gene transfer system and applications to human gene therapy'
- Progress in Nucleic Acid Research and Molecular Biology, Volume 38, issued 1990, J.R. McLACHLIN et al., "Retroviral-Mediated Gene Transfer", pages 91-135, see entire article.
- Journal of Virology, Volume 62, Number 7, issued July 1988, D.D.L. BOWTELL et al., "Comparison of Expression in Hemopoietic Cells by Retroviral Vectors Carrying Two Genes", pages 2464-2473, see entire article.
- Nature, Volume 334, issued 28 July 1988, J. PELLETIER et al., "Internal initiation of translation of eukaryotic mRNA directed by a sequence derived from poliovirus RNA", pages 320-325, see entire article.
- Journal of Virology, Volume 63, Number 1, issued January 1989, J. PELLETIER et al., "Internal Binding of Eucaryotic Ribosomes on Poliovirus RNA: Translation in HeLa Cell Extracts", pages 441-444, see entire article.
- Journal of Virology, Volume 63, Number 4, issued April 1989, S.K. JANG et al., "Initiation of Protein Synthesis by Internal Entry of Ribosomes into the 5' Nontranslated Region of Encephalomyocarditis Virus RNA In Vivo", pages 1651-1660, see entire article.

## Description

This invention relates to the production of a retroviral vector which is capable of expressing multiple protein translation events from a single polycistronic mRNA; without the use of internal promoter elements.

Retroviral vectors are useful as agents to mediate retroviral-mediated gene transfer into eukaryotic cells. Such vectors are generally constructed such that the majority of sequences coding for the structural genes of the virus are deleted and replaced by the gene(s) of interest.

These new genes have been incorporated into the proviral backbone in several general ways. The most straightforward constructions are ones in which the structural genes of the retrovirus are replaced by a single gene which then is transcribed under the control of the viral regulatory sequences within the long terminal repeat (LTR). Retroviral vectors have also been constructed which can introduce more than one gene into target cells. Usually, in such vectors one gene is under the regulatory control of the viral LTR, while the second gene is expressed either off a spliced message (1, 2) or is under the regulation of its own, internal promoter.

Efforts have been directed at minimizing the viral component of the viral backbone, largely in an effort to reduce the chance for recombination between the vector and the packaging-defective helper virus within packaging cells. A packaging-defective helper virus is necessary to provide the structural genes of a retrovirus, which have been deleted from the vector itself.

Gene therapy or drug delivery via gene transfer entails the creation of specialized vectors each vector being applicable only to a particular disease. Thus, it is desirable that a vector cloning system be available which consistently maintains the necessary safety features yet permits maximal flexibility in vector design. Subtle changes in gene position, or in the specific combination of regulatory sequence(s) with the gene of interest, can lead to profound differences in vector titer or in the way that transferred genes function in target cells.

Current vector designs require the use of internal promoter elements in a retroviral vector in order to initiate an internal mRNA. Therefore, when multiple genes were desired in a retroviral vector, it would be necessary to have multiple promoter elements within the one retroviral vector. One potential problem with retroviral vectors containing multiple transcription units is that, it has been observed that if selection is applied for one gene, expression of the other gene can be reduced or lost completely. This has been termed promoter suppression (3,4).

### SUMMARY OF THE INVENTION

The present invention relates to a retroviral expression vector which encompasses a first DNA sequence which encodes for the expression of a first protein and a second DNA sequence which encodes for the expression of a second protein. The vector includes a first promoter sequence for expressing mRNA from from both the first and second DNA sequence and an internal ribosome entry site between the first and second DNA sequence. The promoter sequence, the first DNA sequence, the internal ribosome entry site and the second DNA sequence are operably linked in the retroviral vector to produce a polycistronic mRNA and expression of first and second independent proteins from the mRNA expressed by the promoter sequence.

An embodiment of the present invention provides for the retroviral vector described above wherein the internal ribosome entry site is from a picornavirus.

Gene expression in the picornaviridae famly of viruses is unusual in that their 5' mRNA terminus is pUp...and they possess long untranslated leader sequences (5-6). Analysis of picornaviruses indicate that they are able to bypass the standard ribosome scanning model of translation and begin translation at internal sites (8-11). Internal ribosome entry sites (IRES) have been identified in the long 5' untranslated regions of picornaviruses which can be removed from their viral setting and linked to unrelated genes to produce polycistronic mRNAs (8,10,12).

Embodiments of the present invention relate to operably linking picornavirus IRES elements to various genes and the insertion of these IRES-gene fusions into retroviral vectors which are translated to yield functional gene products. These picornavirus IRES vectors permit several proteins to be produced from a single vector without alternate splicing or multiple transcription units thus eliminating the potential of promoter suppression. Additionally, the coupling or translation of two (or more) different proteins may have significant applications in human gene therapy where the expression in a given cell of multiple heterologous proteins or distinct subunits of a multimeric protein is necessary.

### BRIEF DESCRIPTION OF THE DRAWING

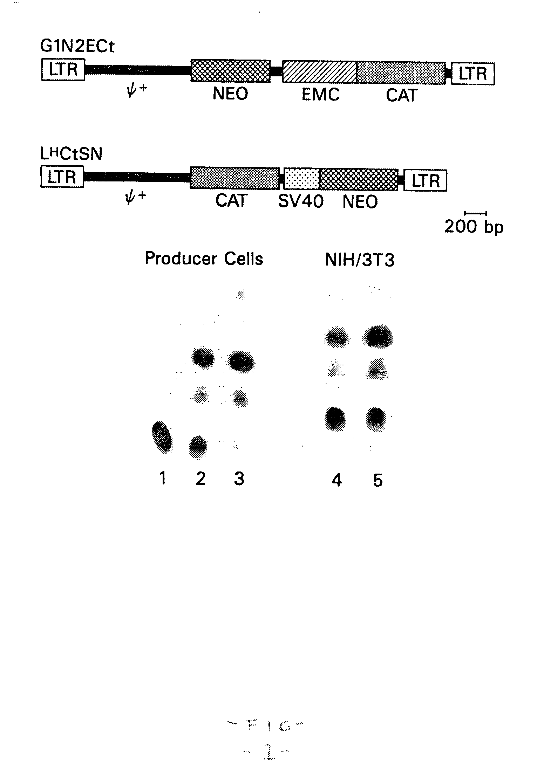

Figure 1. EMC CAT Vector. Shown on the top of the figure is a diagram of the EMC/CAT vector G1N2ECt and the control CAT vector L^{H}CtSN. Below is shown the autoradiograms from CAT enzyme analysis (1 hr. incubation). Lane 1, producer cells transfected with pTM1-CAT; lane 2, G1N2ECt transfected producer cells; lane 3, L^{H}CtSN transfected producer cells; lane 4, G1N2ECt tranduced NIH/3T3 cells; and lane 5, L^{H}CtSN transduced NIH/3T3 cells.

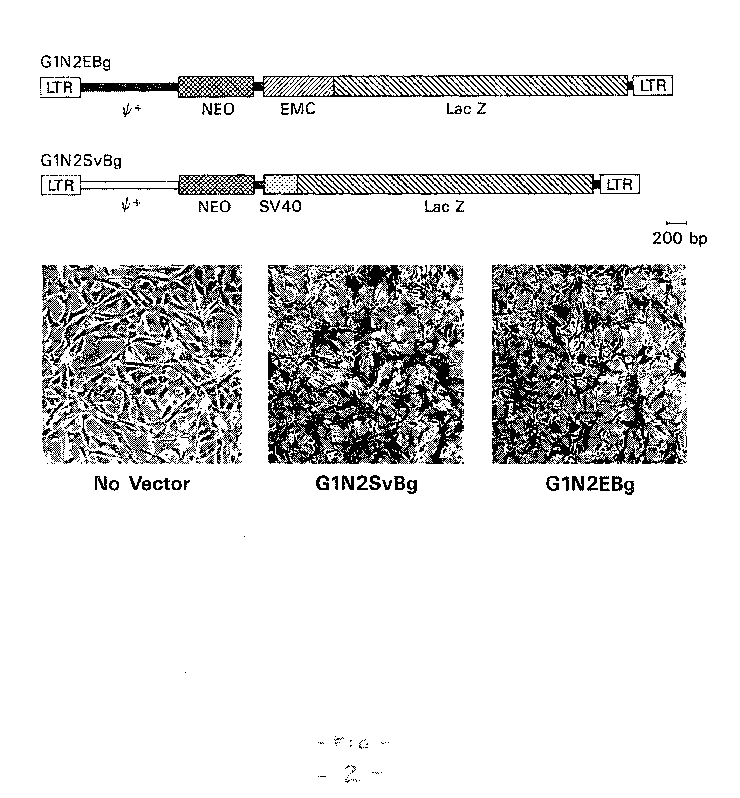

Figure 2. EMC β-GAL Vector. Shown on the top of the figure is a diagram of the EMC/β-gal vector G1N2EBg and the control β-gal vector, G1N2SvBg. Below is shown photomicrographs of in situ stained producer cell lines transfected with the indicated vectors.

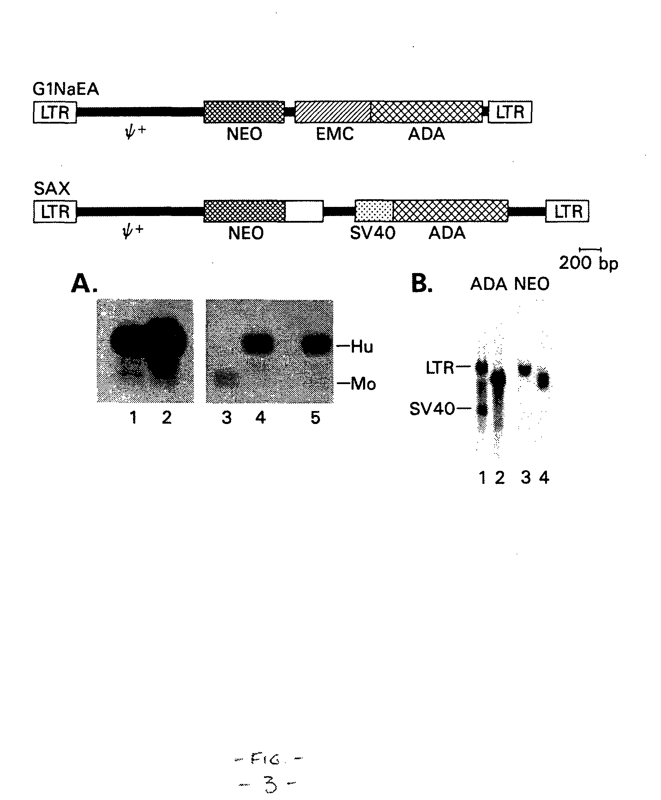

Figure 3. ADA EMC Vector. Shown on the top of the figure is a diagram of the EMC/ADA vector G1NaEA, and the control ADA vector, SAX. Panel A, starch gel analysis for ADA enzyme activity, equal amounts of total cell lysates were used for each sample, the location of the human (Hu) and mouse (Mo) ADA enzymes are indicated. Lane 1, SAX producer cells; lane 2, G1NaEA producer cells; lane 3, NIH/3T3 cells; lane 4, SAX transduced 3T3 cells; and lane 5, G1NaEA transduced 3T3 cells. Panel B, Northern blot analysis using 20 µg of total cell RNA with the indicated probes; ADA, lanes 1 and 2; NEO lanes 3 and 4. Samples were as follows: lanes 1 and 3, RNA from SAX producer cells; lanes 2 and 4, RNA from G1NaEA producer cells. The transcripts originating from the LTR or SV40 promoters are as indicated.

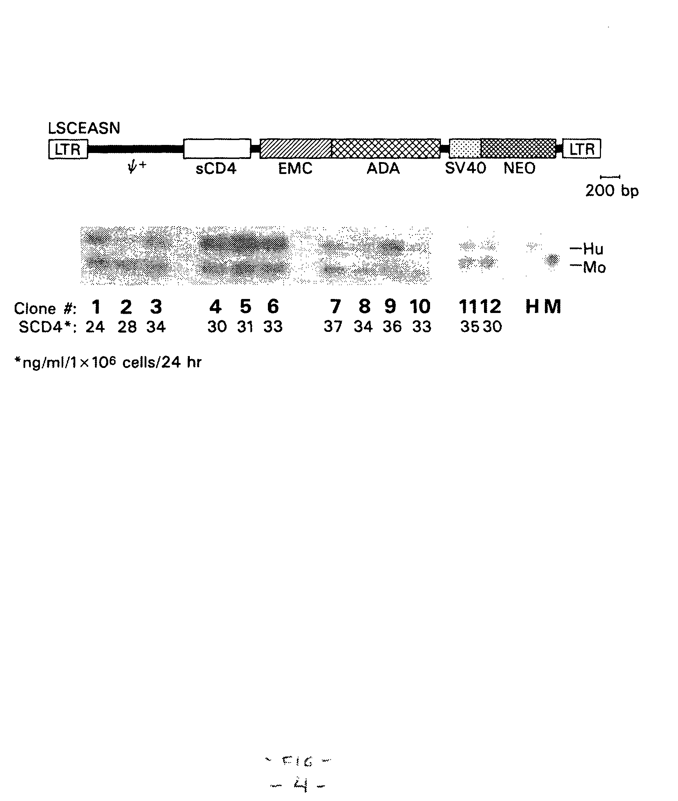

Figure 4. sCD4-ADA-NEO Triple Gene Vector. Shown on the top of the figure is a diagram of the LSCEASN (sCD4,ADA, and NEO) triple gene vector. Below is shown the results of ADA starch gel analysis from 12 G418^{R} producer cell clones (numbers 1-12, H=human control, M=mouse control) and the amounts of sCD4 produced in the culture medium as measured by ELISA.

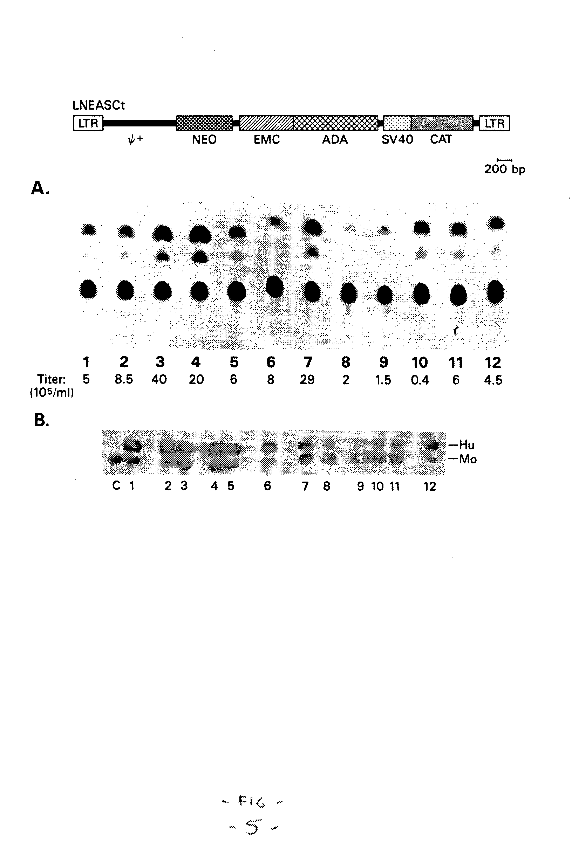

Figure 5. NEO-ADA-CAT Triple Gene Vector. Shown on the top of the figure is a diagram of the LNEASCt (NEO, ADA, and CAT) triple gene vector. Panel A, autoradiogram of resultant CAT activity from 12 G418^{R} producer cell clones (number 1-12), and the titer measured on NIH/3T3 cells of G418^{R} cfu/ml obtained from the producer cells. Panel B, ADA starch gel analysis from the 12 producer cell clones (numbers 1-12), C = NIH/3T3 cells, human (Hu) and mouse (Mo) ADA bands are indicated.

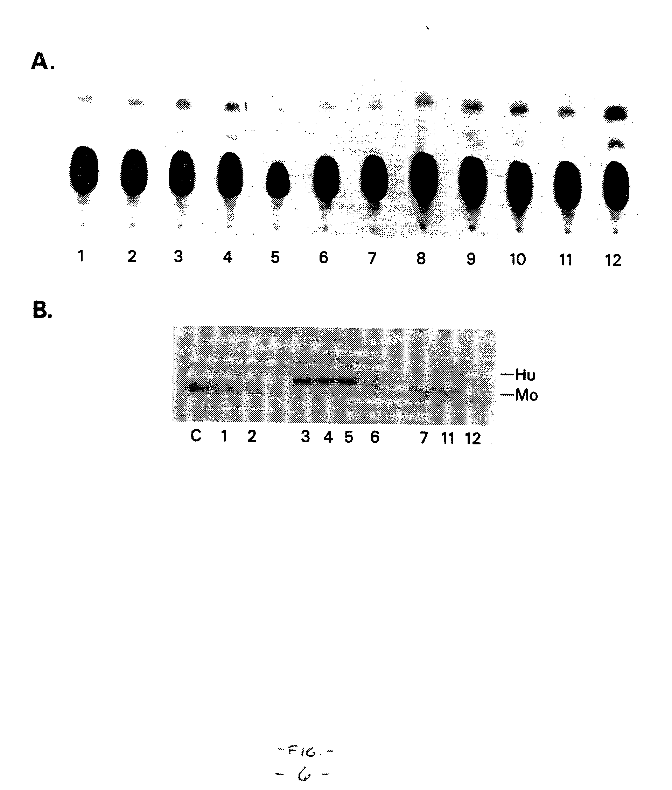

Figure 6. Expression in Triple Gene Transduced Cells. Panel A, resultant autoradiogram of CAT enzyme analysis from 12 NIH/3T3 cell populations transduced and selected with supernatant from the 12 producer cell clones in Figure 5. Panel B, ADA starch gel analysis analysis from 9 NIH/3T3 cell populations transduced and selected with supernatant from the 9 indicated producer cell clones used in Figure 5. Control 3T3 cells were in lane C, the position of the human (Hu) and mouse (Mo) ADA bands are indicated, all human ADA bands were easily visible in the original wet gel.

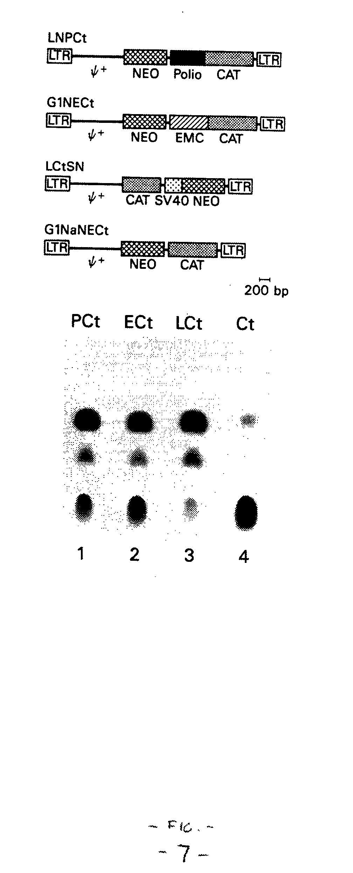

Figure 7. Polio IRES Vector. Shown on the top of the figure are diagrams of the CAT constructs tested in this experiment. Below, autoradiogram of the CAT enzyme analysis (1 hr. incubation) from G418^{R} producer cell lines transfected with lane 1, LNPCt; lane 2, G1NECt, lane 3, LCtSN, and lane 4, G1NaNECt.

### DETAILED DESCRIPTION OF THE INVENTION

It is therefore an object of the present invention to provide a retroviral vector which is capable of expressing multiple genes from a single mRNA using internal ribosome entry sites. It is a further object of the present invention to provide a retroviral expression vector capable of expressing multiple proteins from a host genome.

The retroviral expression vector comprises a first DNA sequence which encodes for the expression of a first protein and a second DNA sequence which encodes for the expression of a second protein. The vector also includes a first promoter sequence for expressing mRNA from both the first and second DNA sequences and picornavirus internal ribosome entry site between the first and second DNA sequences. All of these elements (the promoter sequence, the first DNA sequence, the internal ribosome entry site and the second DNA sequence) are operably linked in the retroviral vector to produce polycistronic mRNA and expression of first and second independent proteins from the mRNA expressed by the promoter.

An embodiment of the present invention provides for the retroviral expression vector described above wherein the internal ribosome entry site is from a picornavirus. An embodiment of this expression vector wherein the picornavirus IRES is from encephalomyocarditis virus, preferably, nucleotide nos. 163 to 746 of the encephalomyocarditis virus.

Another embodiment of this invention provides an expression vector wherein the picornavirus IRES is from poliovirus, preferably, nucleotide nos. 28 to 640 of poliovirus.

The retroviral vector of the present invention may also provide for the first promoter being LTR sequences from a retroviral genome.

Additionally, the present invention provides for a eukaryotic cell, preferably an animal cell, most preferably a human cell(s) which as been genetically engineered using the above retroviral vector. Representative examples of such human cells include, hepatocytes, endothelial cells, bone marrow cells, fibroblasts, etc.

Another object of the present invntion provides a process for producing a retroviral vector capable of producing polycistronic mRNA and capable of expressing at least two independent proteins. The method involves operably linking in a retroviral expression vector a first DNA sequence for encoding the expression of a first protein, a second DNA sequence for encoding the expression of a second protein, a first promoter sequence for expressing mRNA from both the first and second DNA sequences and an picornavirus internal ribosome entry site between said and first and second DNA sequences.

An embodiment of this object of the present invention provides for producing a retroviral vector as identified above wherein the promoter is LTR sequences of a retrovirus genome.

The process described above provides for the internal ribosome entry site derived from a picornavirus. Examples of such picornaviruses include but are not limited to encephalomyocarditis, preferably nucleotide nos. 163-746 and poliovirus, preferably nucleotide Nos 28-640, foot and mouth disease virus preferably nucleotide numbers 369-804.

Additionally the process described above provides for an animal cell which may be genetically engineered using the retroviral vector produced by the above process.

In a preferred embodiment the retroviral expression vector will contain at least one DNA sequence which encodes for the expression of a therapeutic protein. The term therapeutic protein is used in its broadest sense and means any protein or material which has a beneficial effect on the host. The therapeutic protein may be the form of one or more proteins. As representative examples, there may be mentioned: soluble CD-4, Factor VIII, Factor IX, von Willebrand Factor, TPA; urokinase; hirudin; the interferons; tumor necrosis factor, the interleukins, hematopoietic growth factors (G-CSF, GM-CSF, IL3, erythropoietin), antibodies, glucocerebrosidase; adenosine diaminose (ADA); chloramphenicol acetyl transferase (CAT); β-galatosidase (β-gal); phenylalanine hydroxylase, human growth hormone, insulin, etc. The selection of a suitable DNA sequence for a therapeutic protein is deemed to be within the scope of those skilled in the art from the teachings herein.

Many retroviral vectors may be constructed to include the elements of the retroviral vector as claimed herein. Examples of such retroviral vectors are as follows: Moloney leukemia virus, spleen necrosis virus and vectors derived from retroviruses such as Rous sarcoma virus and Harvey sarcoma virus. Specific vectors which may be constructed in accordance with the present invention are described in the examples hereinbelow.

Bender et al., J.Virol. 61:1639-1649 (1987) have described a described a series of vectors, based on the N2 vector (Armentano, et al., J. Virol., 61:1647-1650) containing a series of deletions and substitutions to reduce to an absolute minimum the homology between the vector and packaging systems. These changes have also reduced the likelihood that viral proteins would be expressed. In the first of these vectors, LNL-XHC, there was altered, by site-directed mutagenesis, the natural ATG start condon of gag to TAG, thereby eliminating unintended protein synthesis from that point. In Moloney murine leukemia virus (MoMuLV), 5' to the authentic gag start, an open reading frame exists which permits expression of another glycosylated protein (pPr80^{gag}). Moloney murine sarcoma virus (MoMuSV) has alterations in this 5' region, including a frameshift and loss of glycosylation sites, which obviate potential expression of the amino terminus of pPr80^{gag}. Therefore, the vector LNL6 was made, which incorporated both the altered ATG of LNL-XHC and the 5' portion of MoMuSV. The 5' structure of the LN vector series thus eliminates the possibility of expression of retroviral reading frames, with the subsequent production of viral antigents in genetically transduced target cells. In a final alteration to reduce overlap with packaging-defective helper virus, Miller has eliminated extra env sequences immediately preceding the 3' LTR in the LN vector (15).

Herein applicants have described that picornavirus IRES elements can be linked to various genes and that the gene-IRES fusions, when inserted into retroviral vectors are translated to yield functional gene products. These IRES vectors permit several proteins to be produced from a single vector without alternate splicing or multiple transcriptions units thus eliminating the potential of promoter suppression. Furthermore, the coupling of translation of two (or more) different proteins may have significant applications in human gene therapy where the multimeric protein is necessary.

Additionally, vectors containing mutiple IRES-gene fusions in the same construct using combinations of the EMC and poliovirus IRES elements can be constructed.

In multiple IRES constructs, a single transcription event would generate a polycistronic mRNA that could be translated to yield multiple proteins without utilizing any internal promoters.

Although the retroviral vector is preferably used for therapeutic purposes in the area of gene transfer or gene therapy in humans, the vector may also be employed for transducing cells for in vitro applications; i.e., producing two different proteins in eukaryotic cells.

The coupling of independent protein translations can have several advantages in human gene therapy situations where multiple proteins are needed. An example, of such a situation includes engineering cells to express heterologous proteins which are more efficient at a given task (e.g. reducing the thrombogencity with combinations of TPA plus UPA). It may be further advantageous to have the production of a potentially physiologically dangerous protein be coupled to that of a conditional cell lethal protein (e.g., tumor necrosis factor with herpes virus thymidine kinase).

The following examples are intended to further illustrate that internal ribosome entry sites can be used to produce expression vectors capable of expressing multiple genes, however, the scope of the invention is not intended to be limited thereby.

### MATERIAL AND METHODS

(A) **Molecular Constructs**. All molecular construction techniques used were under standard conditions as described previously (25). G1N2ECt and G1N2EBg vectors, were constructed from the T7 RNA polymerase expression plasmids pOS6 and pOS8 respectively (13, 14); B. Moss, National Institutes of Health, Bethesda, MD). pOS6 was constructed by ligating the 0.8kb NcoI-Bam HI fragment of pT7EMCAt (13) B. Moss, National Institutes of Health, Bethesda, MD) into Nco I-Bam HI sites of the pTM1 vector (26) Bernard Moss, National Institutes of Health, Bethesda, MD). pOS8 was constructed by Eco RI digestion/Klenow fill-in and selfligation, followed by digestion with Bam HI and ligation with the 3.0kb Bam HI fragment of p11X B(Bernard Moss, National Institutes of Health, Bethesda). Cla I plus Bsp MII were used to excise T7-EMC/CAT and T7-EMC/β-gal expression cassettes. The resulting fragments were made blunt ended by Klenow fill-in, and ligated into the Hind III cut/Klenow fill-in site of pG1N2 to produce pG1N2ECt, and pGlN2EBg. To construct an EMC/ADA vector, the EMC IRES was isolated from pTM1 using polymerase chain reaction (PCR; 30 cycles: 92°C, 2 min., 56°C, 2 min., and 37°C, 3 min.) amplification/restriction enzyme site addition (1µM each oligonucleotide primers, 5'-AACGGTTTCCCTCGAGCGGGATCA-3' plus 5'-TTTGTTAGCAGCCGGATCGT-3' in 100µl volume containing 50mM KCl, 10mM Tris-HCl pH8.3, 1.5mM MgCl₂, 0.1% gelatin, 200µM each dNTP, and 2.5U Tag DNA polymerase) to yield a fragment with Xho I ends which was cloned into the Xho I site Bluescript II SK+(Stratagene, La Jolla CA) to yield pEMC-F. PCR (30 cycles: 92°C, 2 min., 56°C, 2 min., and 37°C, 3 min.) was similarly used (1µM each oligonucleotide primers 5'-TGCGAGACCATGGGACAGACGCCC-3' plus 5'-CGGAAGTGTGATCACCTAGGCGAC-3' in 100µl volume containing 50mM KCl, 10mM Tris-HCl pH8.3, 1.5mM MgCl₂, 0.1% gelatin, 200µM each dNTP, and 2.5U Taq DNA polymerase) to produce a fragment containing the ADA gene using the SAX retroviral vector (20) (W. French Anderson, National Institutes of Health, Bethesda, MD) as a template. The ADA fragment was digested with Nco I plus Xba I and cloned into the corresponding sites in pEMC-F to produce pEMCADA. The EMC/ADA fragment was excised by Xba I digestion/Klenow fill-in plus Xho I digestion and ligate to Apa I cut/T4 DNA polymerase fill-in plus Xho I cut retroviral vector pG1Na, to produce pGlNaEA. The starting vectors for the triple gene cosntructs, LSCSN and LNSvCt were produced by inserting the soluble CD4 gene and CAT gene into the Eco RI plus Xho I (for CD4) or Hind III (for CAT) sites of LXSN and LNSC respectively (15) (A. Dusty Miller, Fred Hutchinson Cancer Center, Seattle, WA). The EMCADA fragment was excised from pECADA by Xba I digestion/Klenow fill-in plus Xho I digestion and ligated to Bam HI cut/Klenow fill-in plus Xho I LSCSN to produce LSCEASN. To produce LNEASCt, pEMCADA digested with Xho I plus Xba I, filled in with Klenow and ligated to Bam HI cut/Klenow fill-in LNSvCt. The polio IRES vector was constructed by PCR amplification/restriction site addition (1µM each oligonucleotide primers 5'-CCCAGATCTCCACGTGGCGGC-3' plus 5'-ACCGGAAGGCCTATCCAATTC-3' in 100µl volume containing 50mM KCl, 10mM Tris-HCl pH8.3, 1.5mM MgCl₂, 0.1% gelatin, 200µM each dNTP, and 2.5U Taq DNA polymerase) using pPV16 as a template (7), Bernard Moss, National Institues of Health, Bethesda, MD). The PCR generated a fragment with Bgl II and Stu I ends which was ligated into Bam HI plus Stu I cut LNSvCt to yield LNPCt. The vector G1NECt is similar to G1N2ECt but contains a slighlty different NEO gene. Vector G1NaNECt was constructed by Nco I digestion/Klenow fill-in plus Xho I digestion of pTM1, followed by ligation in to Bam HI cut/Klenow fill-in plus Xho I cut pG1Na. LCtSN and L^{H}CtSN were constructed by ligating a Hind HIII cut/Klenow fill-in CAT fragment into the Hpa I site of LXSN and L^{H}XSN respectively (the L^{H}XSN vector is the same as LXSN but with a substituted U3 region from Harvey murine sarcoma virus (Larry Couture, National Institutes of Health, Bethaesda, MD). The preparation of vectors pG1N2, pG1Na, and pG1N2SvBg is disclosed in Wo91/10728, M. Eglitis, et al.

### CELL CULTURE AND VECTOR PRODUCTION

Retroviral vector producer cell lines were generated by the micro-ping-pong procedure (16,17). In brief, 50 ug of DNA was used to transfect (via calcium phosphate coprecipitation) a mixture of the ecotropic packaging cell line GP+E-86 (23) (Arthur Banks, Columbia University, New York, N.Y.), and the amphotropic packaging cell line PA317 (24) (A.D. Miller, Fred Hutchinson Cancer Center, Seattle, WA). The packaging cell line mixtures are maintained in culture for at least one week to permit vector amplification. Selection for vector integration is obtained by growth in the presence of the neomycin analog G418 (400 ug/ml active concentration). Recombinant retroviral vector preparations were prepared by harvesting cell culture medium from confluent 100mm tissue culture dishes following 24 hr incubation in 10ml fresh culture medium (Dulbecco's modified Eagle's medium containing 10% fetal bovine serum). Cell free supernatants were collected by filtration through 0.22 um filter units and stored at-70°C until use. Transduction of mouse NIH/3T3 cells was conducted by incubation with recombinant viral supernatant containing 8 ug/ml polybrene at 37°C for 2 hr, followed by removal of virus-containing medium and replacement with fresh culture medium. Transduced cell populations were selected by growth in G418 (400 ug/ml) for 10-14 days. Cell clones were obtained using cloning rings following limiting dilution. **GENE EXPRESSION ASSAYS** CAT enzyme assays were performed by first lysing cells (at 4°C) in 0.25M Tris-HCl(pH 7.5)/0.1% NP-40, followed by freezing on dry ice, thawing at 37°C (5 min), heating to 60°C (15 min) and removal of cellular debris by centrifugation (top sped, eppendorf microcentrifuge, 4°C, 5 min). After normalization for equal amounts of protein (Bio-Rad Protein Assay), cell extracts were mixed with acetyl-CoEnzyme A and 14C-chloramphenicol and incubated at 37° C for 1-4 hours as necessary to stay within the linear range of CAT activity. Chloramphenicol and acetylated prducts were extracted with ethyl acetate and applied to thin layer chromatography plates. Chromatographs were run in 95% CHCL₃ 5% methanol. Imaging was obtained by autoradiography and quantitation by direct beta particle counting of the TLC plates on a Betascope 603 instrument. In situ staining for β-galactosidase and assays were performed as described (1 unit of β-gal = the amount of enzyme that hydrolyzes 1 umole ONPG to O-nitrophenol/min at 30°C, pH=7.5) (18). Northern blot analysis was performed on formaldehyde agarose gels using RNA extracted with RNazol (CINNA Biotex, Friendswood, TX). ADA assays were performed on starch gels as described (19). Soluble CD4 levels were measured using a CD4/gpl20 capture ELISA (American Biotechnologies, Cambridge, MA).

**Construction of EMC reporter gene vectors**. To determine if the picornavirus IRES elements could function in a retroviral vector, we transferred two IRES - containing prokaryotic reporter genes from plasmids pOS6(CAT) and pOS8 (β-gal) into NEO-containing retroviral vectors. The two plasmids (pTMl-/CAT pTMl-Bgal) use the IRES from encephalomyocarditis (EMC) virus to increase the translation of mRNAs expressed from a bacteriaophage T7 RNA polymerase transcription unit (13,14). The two reporter gene vectors constructed, G1N2ECt and G1N2EBg, were then introduced into retroviral vector packaging cell lines along with control vector and plasmid DNAs. In the first experiment (Fig.1) we transfected the pTM1-CAT plasmid (lane 1), G1N2ECt (lane 2) or L^{H}CtSN (lane 3) into a PA317/GP+E-86 coculture and expanded the cells in culture for two weeks to allow vector spread. Cell lysates were prepared and equal amounts of protein used to assay for CAT activities as described (see methods). Figure 1 clearly shows significant CAT activity, from the G1N2ECt IRES vector in comparison to the activity driven by the very strong chimeric LTR in L^{H}CSN. No activity is seen in the control cells and CAT expression is dependent on the presence of an IRES element (Fig. 7). Quantitation of CAT activity, performed in the linear range, indicated that G1N2ECt containing cells produce 55% of the L^{H}CtSN activity. To rule out the possibility that the EMC IRES was some how serving as a promoter element in the context of a retroviral vector, a construct with the EMC/CAT fusion in the reverse orientation was produced and tested. No CAT activity was observed from the reverse orientation EMC/CAT vector (data not shown).

Retroviral vector containing supernatant from the G1N2ECt and L^{H}CtSN producer cells was then used to transduce NIH/3T3 cells. Following transduction, the cells were cultured for five days and then harvested for CAT assays. The CAT activity for G1N2ECt transduced 3T3 cells (lane 4) and for L^{H}CtSN transduced 3T3 cells (lane 5) is shown in Figure 1. The data indicate that the G1N2ECt IRES vector can produce a functional retroviral vector particles that can productively transfer and express a IRES/reporter gene in an appropriate target cells.

Next, we constructed a retroviral vector containing the EMC IRES linked to the β-galactosidase reporter gene. The G1N2EBg vector was transfected into a packaging cell line coculture with pTM1-ßGal and G1N2SvBg serving as controls. Following a two week culture to permit vector spread, the producer cells were assayed for β-galactosidase activity by an in situ enzyme activity assay. Figure 2 shows numerous blue staining cells in the G1N2EBg and the positive control G1N2SvBg cultures with no staining cells in the pTM1-ßgal negative control culture.
Retroviral-vector-containing producer cell supernatant was then used to transduce NIH/3T3 cells. The cells were harvested 5 days post transduction the cells were harvested and assayed for β-gal activity. Functinal transfer to 3T3 cells of the β-galactosidase enzyme activity was detected by in situ staining (data not shown) and then quantitated by measuring β-gel enzyme activity in cell extracts; which was shown to be 7.2 x 10⁴U/mg for G1N2EBg and 9.5 x 10⁴U/mg for G1N2SvBg.
**Construction of an EMC human ADA vector.** To evaluate the use of IRES elements in the construction of retroviral vectors for potential human gene therapy applications, a fusion between the EMC IRES and the human adenosine deaminase (ADA) gene was assembled and introduced into a retroviral vectors. A DNA fragment containing the EMC IRES was synthesized via polymerase chain reaction (PCR) amplification with the addition of convenient cloning sites, and used to generate a plasmid (pEMC-F) which contains the EMC IRES without flanking T7 RNA polymerase transcription signals. The human ADA gene was then synthesized, again using PCR, and cloned into the EMC plasmid to generate pEMCADA. The EMC/ADA fusion was excised from pEMCADA and inserted into the retroviral vector G1Na yielding G1NaEA (Fig.3).

DNA for the G1NaEA vector and the control ADA vector SAX (20), were then used to generate retroviral producer cell lines. Producer cell cocultures were grown for 1 week in standard culture medium and then selected for stable vector integration by culture for 2 weeks in the presence of the neomycin analog G418. The selected (G418^{R}) producer cell populations were then used to generate vector containing supernatant for titer determinations, and subjected to gene expression analysis.

Figure 3, panel A shows the results of ADA starch gel analysis on the G1NaEA producer cells (lane 2) and SAX control producer cells (lane 1), both producer cell populations make large amounts of human ADA. Northern blot analysis (Fig.3, panel B), was then used to visualize the RNA transcripts from the two vectors. For SAX, a full length LTR transcript as well as the internal SV40 transcript are seen with the ADA probe while only the full length transcript is observed with the neo probe (lanes 1 and 3). In the case of G1NaEA, only one full length transcript is identified by Northern blot analysis with either the ADA or NEO probe (lanes 2 and 4).

Retroviral vector-containing supernatant from the producer cell populations were then used to transduce NIH/3T3 cells as well as determine the vector titer on 3T3 cells. Both producer cell populations yielded good titer vector supernatants with SAX being 1.9 x 10⁶ G418^{R} cfu/ml and G1NaEA being 1.2 x 10⁶ G418^{R} cfu/ml. The G418^{R} 3T3 cells were next assayed for ADA activity. ADA starch gel analysis demonstrated functional transfer of the human ADA gene into the 3T3 cells by the G1NaEA IRES vector (Fig.3, panel A, lane 5). In this experiment, the 3T3 cells transduced with control SAX vector produced only slightly less human ADA than the IRES vector (Fig.3, panel, lane 4).

**Construction of triple gene vectors**. To test the versatility of IRES elements in the construction of complex retroviral vectors, we inserted the EMC/ADA fusion gene into two independent double gene vectors to generate three gene vectors. The first receipient vector LSCSN uses the LTR to promote the expression of the anti-HIV agent soluble CD4 (sCD4) (21) and an internal SV40 early region promoter to drive the NEO selectable marker gene (21). The EMC/ADA fragment was introduced after the sCD4 translation stop codon and 5' to the start of the SV40 promoter to generate LSCEASN (Fig.4). LSCEASN DNA was transfected into a packaging cell line coculture which was grown for one week before being passaged, at limiting dilution, into G418-containing medium. Twelve G418^{R} producer cell clones synthesize both the human ADA enzyme and produce the sCD4 protein (Fig. 4).

The second two-gene retroviral vector used as recipient for the EMC/ADA fragment was LNSCt, a vector which uses the LTR to drive NEO expression and has an internal SV40 promoter directing CAT expression. EMC/ADA was inserted 3' to the NEO gene stop codon and upstream of the SV40 promoter to generate LNEASCt (Fig.5). LNEASCt DNA was transfected into packaging cells cultured for one week, and then G418^{R} producer cell clones were isolated by limiting dilution. Twelve producer cell clones were expanded and used to isolate vector containing supernatant to determine G418^{R} titer, and analyzed for both CAT and ADA gene expression. Figure 5 shows that all twelve producer cell clones had both CAT (panel A) and human ADA (panel B) enzyme activity. The titer from the twelve clones ranged from 4 x 10⁴ G418^{R} cfu/ml for clone 10 to 4 x 10⁶ G418^{R} cfu/ml for clone 4.

Retroviral vector-containing supernatant from each of the twelve LNEASCt producer cell clones was then used to transduce NIH/3T3 cells. Twelve G418R 3T3 cell cultures were expanded and assayed for CAT and human ADA enzyme activity. CAT activity was documented in 12 of 12 3T3 cell cultures (Fig.6, panel A) and human ADA activity observed in 9 out of 9 tested cultures (Fig. 6, panel B). In this particular series of transcuctions, both CAT and human ADA enzyme activities were noticeably lower in 3T3 cells than in producer cells (Fig.5). Analysis of CAT activity in 3T3 cells generated using the parent two gene vector LNSCt showed similar CAT activity, suggesting that the particular SV40/CAT internal gene in this vector is not very active (data not shown).
**Construction of a Polio IRES vector**. In the next series of experiments, we isolated the IRES from poliovirus and used it to construct a retroviral vector. PCR was used to generate a fragment contain the 600 bp IRES element from the 5' untranslated region of poliovirus (Mahoney strain). The polio IRES was then inserted 3' to the NEO stop codon and upstream of a CAT reporter gene to generate LNPCt. This vector along with a similar EMC IRES construct (GINECt), a LTR driven CAT positive control vector (LCSN), and vector containing CAT but no IRES sequences (G1NaNECt) were transfected into packaging cell cocultures. The cultures were grown for one week in standard medium and then selected for vector containing cells by growth for two weeks in G418 containing culture medium. Completely selected cultures were then harvested and assayed for CAT enzyme activity (Fig.7). The data from Figure 7 indicate that the polio IRES functions as well as (if not slightly better than) the EMC IRES. Both IRES vectors compared favorably with the CAT activity driven by the strong LTR promoter (LNPCt 70% and G1NECt 50% of LCtSN). A small amount of CAT activity is seen in the construct without an IRES element (GlNaENCt). This limited activity may be due to initiation at internal AUG codons, as has been previously reported in retroviral vectors (22) and the mechanism of this leaky expression is under investigation.

While the present invention has been described in conjunction with specific embodiments thereof, it is evident that many alternatives, modifications and variations will be apparent to those skilled in the art in view of the foregoing description. Accordingly, the invention is intended to embrace all such alternatives, modifications and variations in following within the broadest scope and spirit of the following claims.

Numerous modifications and variations of the present invention are possible in light of the above teachings, and are therefore, within the scope of the Appended Claims, the invention may be practiced otherwise than as particularly described.

### References

1. Cepko, C.L., Roberts, B.E., and Mulligan, R.C. (1984). Cell 37, 1053-1062.
2. Bowtell, d.D.L., et al. J.Virol. (1988). 62,2464-2473.
3. Emerman, M. and Temin, H.M. (1984). Cell 39, 459-467.
4. Emerman, M. and Temin, H.M. (1986). Mol. Cell. Biol. 6, 792-800.
5. Hewellett et al. (1976) PNAS, 73, 327-330.
6. Nomoto, et al. (1976). PNAS, 73, 375-380.
7. Van-Der Werf, et al. (1986). Proc. Natl. Acad. Sci. USA 83, 2330-2334.
8. Pelletier, J. and Sonenberg, N. (1988). Nature, 334, 320-325.
9. Jang, S.K. et al. (1988). J. Virol. 62, 2636-2643.
10. Pelletier, J., and Sonenberg, N. (1989). J.Virol. 63, 441-444.
11. Jang, S.K., and Wimmer, E. (1990). Genes and Development. 4, 1560-1572.
12. Jang, S.K., et al. (1989). J.Virol. 63, 1651-1660.
13. Elroy-Stein, O., Fuerst, T.R., and Moss, B. (1989). Proc. Natl. Acad. USA 86,6126-6130.
14. Elroy-Stein, O., and Moss, B. (1990). Proc. Natl. Acad. Sci. USA 87, 6743-6747..
15. Miller, A.D., et al. (1989). Biotechniques 7,989-980.
16. Bestwick, R.K. et al. (1988). PNAS 85, 5404-5408.
17. Muenchau D. et al. (1990). Virology 176, 262-265.
18. Rosenthal, N. (1987). In, Methods in Enzymology. Eds. Berger, S.L. and Kimmel, A.R. (Academic Press, New York, N.Y.) Vol. 152, pp. 704-720.
19. Lim, B. et al. (1987). Mol. Cell. Biol. 7,3458-3465.
20. Kantoff P.E., et al. (1986). Proc. Natl. Acad. Sci. USA. 83, 6563-6567.
21. Morgan, R.a., et al. (1990). AIDS Res. and Human Retroviruses 6, 183-191.
22. Bandyopadhyay, P.K. and Temin, H.M. (1984). Mol. Cell Biol. 4, 743-748.
23. Markowitz, D., et al. (1988). J. Virol. 62, 1120-1124.
24. Miller, A.D., et al. (1986). Mol. Cell. Biol. 6, 2895-2902.
25. Maniatis, T., et al. (1982). In, Molecular Cloning: A Laboratory Manual, Cold Spring Harbor Laboratory, Cold Spring Harbor, N.Y.
26. Moss, B., et al., (1990). Nature. 348, 91-92.

## Claims

1. A retroviral expression vector capable of expressing multiple proteins from a host genome having incorporated therein:
a first DNA sequence encoding a first protein to be expressed,
a second DNA sequence encoding a second protein to be expressed,
a first promoter sequence for expressing mRNA from both the first and second DNA sequence and a picornavirus internal ribosome entry site between said first and second DNA sequences, said promoter sequence, the first DNA sequence, the internal ribosome entry site and the second DNA sequence being operably linked in said retroviral vector to produce polycistronic mRNA and expression of first and second independent proteins from said mRNA expressed by said promoter.

2. A retroviral expression vector as in claim 1 wherein said promoter is comprised of LTR sequences from a retroviral genome.

3. A retroviral expression vector as in claim 1, wherein said picornavirus internal ribosome entry site is from encephalomyocarditis virus.

4. A retroviral expression vector as in claim 3, wherein said encephalomyocarditis virus internal ribosome entry site is from a portion of the encephalomyocarditis virus 5' untranslated region.

5. A retroviral expression vector as in claim 1, wherein said picornavirus internal ribosome entry site is from poliovirus.

6. A retroviral expression vector as in claim 5, wherein said poliovirus internal ribosome entry site is from a portion of the poliovirus 5' untranslated region.

7. A packaging cell transfected with the retroviral expression vector of claim 1.

8. A packaging cell transfected with the retroviral expression vector of claim 5.

9. A process for constructing a retroviral vector capable of producing polycistronic mRNA and expressing two independent proteins, comprising:
operably linking in a retroviral expression vector a first DNA sequence encoding a first protein to be expressed, a second DNA sequence encoding a second protein to be expressed, a first promoter sequence for expressing mRNA from both the first and second DNA sequences and a picornavirus internal ribosome entry site between said first and second DNA sequences.

10. The process for constructing a retroviral vector according to claim 9, further comprising the steps of transfecting said retroviral expression vector into a packaging cell line and recovering said retroviral vector capable of producing polycistronic mRNA and expressing two independent proteins from supernatant of said transfected packaging cell line.

11. A process as in claim 9 or 10, wherein said promoter is comprised of LTR sequences from a retroviral genome.

12. A process as in claim 9 or 10, wherein said picornavirus internal ribosome entry site is from encephalomyocarditis.

13. A process as in claim 12, wherein said encephalomyocarditis internal ribosome entry site is from a portion of the encephalomyocarditis virus 5' untranslated region.

14. A process as in claim 9 or 10, wherein said picornavirus internal ribosome entry site is from poliovirus.

15. A process as in claim 14, wherein said poliovirus internal ribosome entry site is from a portion of the poliovirus 5' untranslated region.

16. A retroviral vector obtainable from the process of claims 9 or 10.

17. An animal cell transduced with the retroviral vector produced from the process of claim 9 or 10.

18. A human cell transduced with the retroviral vector produced from the process of claim 9 or 10.

## Patentansprüche

1. Retroviraler Expressionsvektor, der zur Expression von multiplen Proteinen von einem Wirtsgenom fähig ist, der folgendes aufweist:
eine erste DNA Sequenz, die für ein erstes zu exprimierendes Protein kodiert,
eine zweite DNA Sequenz, die für ein zweites zu exprimierendes Protein kodiert,
eine erste Promotorsequenz zur Expression von mRNA sowohl von der ersten als auch zweiten DNA Sequenz und
eine interne Ribosomeneintrittsstelle des Picornavirus zwischen der ersten und der zweiten DNA Sequenz, wobei die Promotorsequenz, die erste DNA Sequenz, die interne Ribosomeneintrittsstelle und die zweite DNA Sequenz in diesem retroviralen Vektor so operativ verbunden sind, daß eine polycistronische mRNA gebildet wird und eine Expression der ersten und zweiten unabhängigen Proteine von dieser mRNA stattfindet, die durch diesen Promotor exprimiert wird.

2. Retroviraler Expressionsvektor nach Anspruch 1, worin der Promotor aus LTR Sequenzen aus einem retroviralen Genom besteht.

3. Retroviraler Expressionsvektor nach Anspruch 1, worin die interne Ribosomeneintrittsstelle des Picornavirus vom Encephalomyocarditisvirus stammt.

4. Retroviraler Expressionsvektor nach Anspruch 3, worin die interne Ribosomeneintrittsstelle des Encephalomyocarditisvirus von einem Teil der 5'-untranslatierten Region des Encephalomyocarditisvirus stammt.

5. Retroviraler Expressionsvektor nach Anspruch 1, worin die interne Ribosomeneintrittsstelle des Picornavirus von einem Poliovirus stammt.

6. Retroviraler Expressionsvektor nach Anspruch 5, worin die interne Ribosomeneintrittsstelle des Poliovirus von einem Teil der 5'-untranslatierten Region des Poliovirus stammt.

7. Verpackungszelle, die mit dem retroviralen Expressionsvektor nach Anspruch 1 transfiziert ist

8. Verpackungszelle, die mit dem retroviralen Expressionsvektor nach Anspruch 5 transfiziert ist.

9. Verfahren zur Konstruktion eines retroviralen Vektors, der zur Bildung einer polycistronischen mRNA fähig ist und zwei unabhängige Proteine exprimiert, **gekennzeichnet durch**
operatives Verbinden einer ersten DNA Sequenz, die für ein erstes zu exprimierendes Protein kodiert, einer zweiten DNA Sequenz, die für ein zweites zu exprimierendes Protein kodiert, einer ersten Promotorsequenz zur Expression der mRNA sowohl der ersten als auch der zweiten DNA Sequenz und einer internen Ribosomeneintrittsstelle des Picornavirus zwischen der ersten und der zweiten DNA Sequenz in einem retroviralen Expressionsvektor.

10. Verfahren zur Konstruktion eines retroviralen Vektors nach Anspruch 9, das ferner die Schritte der Transfektion dieses retroviralen Expressionsvektors in eine Verpackungszellinie und Gewinnen dieses retroviralen Vektors umfaßt, der zur Bildung der polycistronischen mRNA und zur Expression zweier unabhängiger Proteine in den Überstand dieser transfizierten Verpackungszellinie fähig ist.

11. Verfahren nach Anspruch 9 oder 10, worin der Promotor aus LTR Sequenzen eines retroviralen Genoms stammt.

12. Verfahren nach Anspruch 9 oder 10, worin die interne Ribosomeneintrittsstelle des Picornavirus vom Encephalomyocarditisvirus stammt.

13. Verfahren nach Anspruch 12, worin die interne Ribosomeneintrittsstelle des Encephalomyocarditisvirus von einem Teil der 5'-untranslatierten Region des Encephalomyocarditisvirus stammt.

14. Verfahren nach Anspruch 9 oder 10, worin die interne Ribosomeneintrittsstelle des Picornavirus von einem Poliovirus stammt.

15. Verfahren nach Anspruch 14, worin die interne Ribosomeneintrittsstelle des Poliovirus von einem Teil der 5'-untranslatierten Region des Poliovirus stammt.

16. Retroviraler Vektor, der nach dem Verfahren von Anspruch 9 oder 10 erhalten werden kann.

17. Tierzelle, die mit einem retroviralen Vektor transduziert ist, der durch das Verfahren von Anspruch 9 oder 10 hergestellt wurde.

18. Humanzelle, die mit einem retroviralen Vektor transduziert ist, der durch das Verfahren von Anspruch 9 oder 10 hergestellt wurde.

## Revendications

1. Vecteur d'expression rétroviral capable d'exprimer plusieurs protéines à partir d'un génome d'hôte dans lequel ont été incorporés :
une première séquence d'ADN codant pour une première protéine à exprimer,
une seconde séquence d'ADN codant pour une seconde protéine à exprimer,
une première séquence promotrice destinée à exprimer un ARNm à partir des deux première et seconde séquences d'ADN,
et un site d'entrée de ribosomes interne de picornavirus entre lesdites première et seconde séquences d'ADN,
ladite séquence promotrice, la première séquence d'ADN, le site d'entrée de ribosomes interne et la seconde séquence d'ADN étant liés de manière opérationnelle dans ledit vecteur rétroviral pour produire un ARNm polycistronique et l'expression d'une première protéine et d'une seconde protéine indépendantes à partir dudit ARNm exprimé par ledit promoteur.

2. Vecteur d'expression rétroviral selon la revendication 1 dans lequel ledit promoteur est composé de séquences LTR venant d'un génome rétroviral.

3. Vecteur d'expression rétroviral selon la revendication 1 dans lequel ledit site d'entrée de ribosomes interne de picornavirus provient du virus de l'encéphalomyocardite.

4. Vecteur d'expression rétroviral selon la revendication 3 dans lequel ledit site d'entrée de ribosomes interne de virus d'encéphalomyocardite provient d'une portion de la région non traduite en 5' du virus de l'encéphalomyocardite.

5. Vecteur d'expression rétroviral selon la revendication 1 dans lequel ledit site d'entrée de ribosomes interne de picornavirus provient du poliovirus.

6. Vecteur d'expression rétroviral selon la revendication 5 dans lequel ledit site d'entrée de ribosomes interne de poliovirus provient d'une portion de la région non traduite en 5' du poliovirus.

7. Cellule d'empaquetage transfectée avec le vecteur d'expression rétroviral de la revendication 1.

8. Cellule d'empaquetage transfectée avec le vecteur d'expression rétroviral de la revendication 5.

9. Procédé de construction d'un vecteur rétroviral capable de produire un ARNm polycistronique et d'exprimer deux protéines indépendantes, comprenant les étapes de :
liaison opérationnelle, dans un vecteur d'expression rétroviral, d'une première séquence d'ADN codant pour une première protéine à exprimer, d'une seconde séquence d'ADN codant pour une seconde protéine à exprimer, d'une première séquence promotrice destinée à exprimer un ARNm à partir des deux première et seconde séquences d'ADN, et d'un site d'entrée de ribosomes interne de picornavirus entre lesdites première et seconde séquences d'ADN.

10. Procédé de construction d'un vecteur rétroviral selon la revendication 9, comprenant en outre les étapes de transfection dudit vecteur d'expression rétroviral dans une lignée cellulaire d'empaquetage, et de récupération dudit vecteur rétroviral capable de produire un ARNm polycistronique et d'exprimer deux protéines indépendantes à partir du surnageant de ladite lignée cellulaire d'empaquetage transfectée.

11. Procédé selon la revendication 9 ou 10 dans lequel ledit promoteur est composé de séquences LTR venant d'un génome rétroviral.

12. Procédé selon la revendication 9 ou 10 dans lequel ledit site d'entrée de ribosomes interne de picornavirus provient du virus de l'encéphalomyocardite.

13. Procédé selon la revendication 12 dans lequel ledit site d'entrée de ribosomes interne de virus d'encéphalomyocardite provient d'une portion de la région non traduite en 5' du virus de l'encéphalomyocardite.

14. Procédé selon la revendication 9 ou 10 dans lequel ledit site d'entrée de ribosomes interne de picornavirus provient du poliovirus.

15. Procédé selon la revendication 14 dans lequel ledit site d'entrée de ribosomes interne de poliovirus provient d'une portion de la région non traduite en 5' du poliovirus.

16. Vecteur rétroviral pouvant être obtenu à partir du procédé des revendications 9 ou 10.

17. Cellule animale transduite avec le vecteur rétroviral produit à partir du procédé de la revendication 9 ou 10.

18. Cellule humaine transduite avec le vecteur rétroviral produit à partir du procédé de la revendication 9 ou 10.
